# EUROPEAN PATENT SPECIFICATION

(11) **EP 1 825 878 B1**
(45) Date of publication and mention of the grant of the patent: **05.04.2017**
(21) Application number: 06075399.3
(22) Date of filing: 20.02.2006
(51) Int. Cl.: A61J 1/06, A61M 5/14, A61M 5/315

(54) **System for administering a medicament comprising a plunger with a passage for flushing**
Medikamentenabgabevorrichtung mit einem Kolben mit einer Passage zum Spülen
Dispositif pour administrer un médicament avec un piston avec une passage pour rincage

(43) Date of publication of application: 29.08.2007
(73) Proprietor: Comecer Netherlands B.V., 8501 XC Joure (NL); B.V. Cyclotron Vrije Universiteit, 1081 HV Amsterdam (NL)
(72) Inventor: Van der Jagt, Pieter Jan, 1083 DC Amsterdam (NL); Quarles van Ufford, Joan, 8517 HC Scharsterburg (NL)
(74) Representative: Verdijck, Gerardus

(56) References cited:
- GB-A- 1 129 643
- US-A- 3 911 916
- US-A- 4 060 082
- US-A- 4 685 910
- US-A- 4 702 737
- US-A- 4 767 415
- US-A- 4 834 705
- US-A- 5 545 139
- US-A1- 2003 236 503

## Description

The present invention relates to an assembly in general and a container in particular for administering a dose of active material, for example a radio active substance containing fluid or a contrast medium fluid for imaging purpose. The assembly comprises a container and a conduit configuration. The conduit configuration is, on the one hand, connectable or at least in use connected to the exit of the container, and, on the other hand, to the patient. An exit is a part of the container. The container has a distal ejection side with said exit and a proximal control side. Further, the container comprises a plunger, being selectively movable, at least in the direction from the proximal to the distal side of the container to eject the dose, which is then beforehand pre-filled in the container, from the exit.

Such assemblies are generally known, and the containers as described above also. Where, in the following, reference is made to any fluid, any type of fluid is in fact meant, where contact therewith or exposure thereto relative to (medical) personnel, who regularly handle or manipulate such fluids, is preferably limited as much as possible.

As an example, reference is made here to a product of the company "Swiss Medical Care". The prior art assembly is referred to as the "auto flush single chamber". This auto flush single chamber is in a fact no more than a syringe, wherein the exit is formed by a needle. The intermediate space between the needle and the hand operated plunger is divided in two compartments by a second plunger, comprising a non-return valve. The compartment between the hand operated plunger and the second plunger is, in use, filled with flushing fluid, whereas the compartment between the needle exit and the second plunger is, in use, to be filled with active material. How the flushing fluid is to be introduced onto the known product, is unknown, unforeseeable and it is doubted that this can be done without exposure of personnel to the active substances containing fluid(s).

Further, when a force is exerted on the manually operated plunger to eject from the needle exit the active material, a operator might exert too high a force, causing the non return valve in the second plunger to open, whereby the flushing medium fluid is released too soon, i.e. before all of the active material is ejected. Further, as a disadvantage of the prior known art of this auto flush single chamber, it is very difficult and cumbersome to fill the compartment between the manually operated and the second plunger with flushing fluid, since a non return valve is arranged in the second plunger, so that no use can be made of the needle exit for also filling the syringe with the flushing fluid.

GB 1,129,643 discloses an injection cartridge comprising a body that on one end is closed by a stopper and on the other end is provided with a piston. Between the stopper and piston is positioned an additional piston, dividing the cartridge into two compartments for two incompatible compositions. The stopper and additional piston can be pierced by a needle. In use the stopper is pierced and a plunger is brought to bear on the piston on the other end of the cartridge.

US 4,834,705 discloses a drug dispensing system comprising a delivery tube, a reservoir at a proximal end in communication with the delivery tube for the infusion fluid, a piston mounted in the cylinder and means such as a one-way valve in the piston or bypass duct in the cylinder to communicate a proximal side of the piston with a distal side of the piston for passage of the fluid.

The present invention has for an object to at least diminish and preferably obviate the above stated drawbacks of the prior art. To this effect, an assembly according to the present invention is distinguished over the above identified prior art in that the plunger comprises a passage for the flushing fluid, which passage is normally closed and selectively opened, and wherein the assembly further comprises an opener, which is arrangeable or at least in use arranged at the exit to act on the plunger to open the passage with the plunger moved to a position in proximity of the exit.

With an assembly according to the present invention, the flushing fluid is, with more certainty, only flushed through the conduit configuration after the plunger has reached the ultimate position thereof in the proximity of the exit, without any risk of flushing fluid being released into the active material before the plunger has reached the exit.

It is noted, that when using an assembly according to the invention, preferably the conduit configuration is arranged in connection with a patient first. Then the container is connected to the conduit configuration. Thus most if not practically all risk of the fluid coming into contact with the environment of the assembly, specifically the handling or manipulating personnel is or can be limited or even obviated.

It is noted, that the present invention also specifically relates to the container as defined in present claim 1, if and in so far as suitable to the employed in an assembly as described herein above.

The exit comprises an exit septum, which is pierceable or at least in use pierced by a needle, by which the conduit configuration is easily connected to the container. Thus the configuration is reversed, wherein the container itself forms a syringe, because in stead of a syringe at the exit of the container in the present embodiment an exit septum is utilised.

Further, an assembly according to the present invention comprises an opener formed by a needle and wherein the passage comprises a passage septum for selective piercing by the opener needle. A septum is a very simple and well suitable embodiment for the passage in the plunger, which can only be opened in cooperation with the opener, when formed by the opener needle.

In particular, an embodiment exhibiting an exit septum as well as a needle forming the opener and an passage septum for piercing also by the opener needle, a very simple and useful embodiment can exhibit the feature, that a single needle is arrangeable or at least in use is arranged to pierce both the exit septum and the passage septum. In such an embodiment the same needle can be used for piercing the passage septum as well as the exit septum, where the passage septum is preferably only pierced by the single needle, when the plunger has reached the proximity of the exit. In such an embodiment further, the single needle can be connected to the conduit configuration. In such an embodiment, the conduit configuration is easily connected to the container, especially at the exit thereof, by inserting the single needle that is connected to the conduit configuration, into the exit septum to await arrival of the plunger with the passage septum oriented toward the single needle. Thus, the flushing fluid can be released from the container with certainty only after the plunger has reached the proximity of the exit.

In the present invention, the proximal control side of the container comprises a closure. Such a closure can be used to form a chamber or compartment at the proximal control side for containing the flushing fluid. Thus, the container can contain in separate compartments the flushing fluid, as well as the active material, with the plunger there between. As such, a self contained unit can be provided, which is pre-filled with flushing fluid on the one side and active material on the other.

In the container with a closure at the proximal control side, the closure comprises a closure septum. Thus, flushing fluid can easily be brought into the compartment at the proximal control side of the container. Further, additional introduction of flushing fluid into this compartment is also possible in a simple and elegant manner.

Further, in the present invention, the proximal control side of the container is connectable or at least in use is connected to a flushing fluid supply. More flushing fluid can be introduced through the conduit configuration, than the amount of flushing, that can beforehand be arranged in any compartment of the container.

In the invention exhibiting a closure septum, as well as a possibility for connecting the container to a flushing fluid supply, the flushing fluid supply comprises preferably a needle for piercing or at least in use actually piercing the closure septum. the invention replenishment of a supply of flushing fluid inside the container is easily possible, as well as providing a sufficient amount of flushing fluid for effectively the conduit configuration to insure, that no residual active material remains therein.

Use is made of a flushing fluid supplied to be connected to the proximal control side of the container and where the flushing fluid supply comprises a needle for piercing a closure septum at the proximal control side, the assembly can, according to the present invention, exhibit yet another feature, wherein the flushing fluid supply comprises a pressure exhorting component, such as a flushing fluid filled syringe, for selectively inserting flushing fluid into the container at the proximal control side thereof and for thereby selectively moving the plunger. According to the present invention, moving the plunger is effected from the proximal control side by manipulating the flushing fluid supply to exhort a power or pressure on the plunger. Thereby easy and elegant handling can be provided.

The present invention will be further elucidated by reference to the enclosed drawings, in which the same or similar components, elements and parts are designated by the same reference numbers, and wherein:
Fig. 1 shows schematically a sequence of steps for manufacturing a container according to the present invention, at least one embodiment thereof;
Fig. 2A-2B schematically show preparation steps prior to use of the container of fig. 1 in an assembly according to the present invention;
Fig. 3A-3F schematically show a succession of steps involving the container in an assembly in use in an embodiment of the present invention;
Fig. 4 shows schematically another container in an assembly, which is not according to the present invention; and
Fig. 5 shows another embodiment of a container and for an assembly according to the present invention.

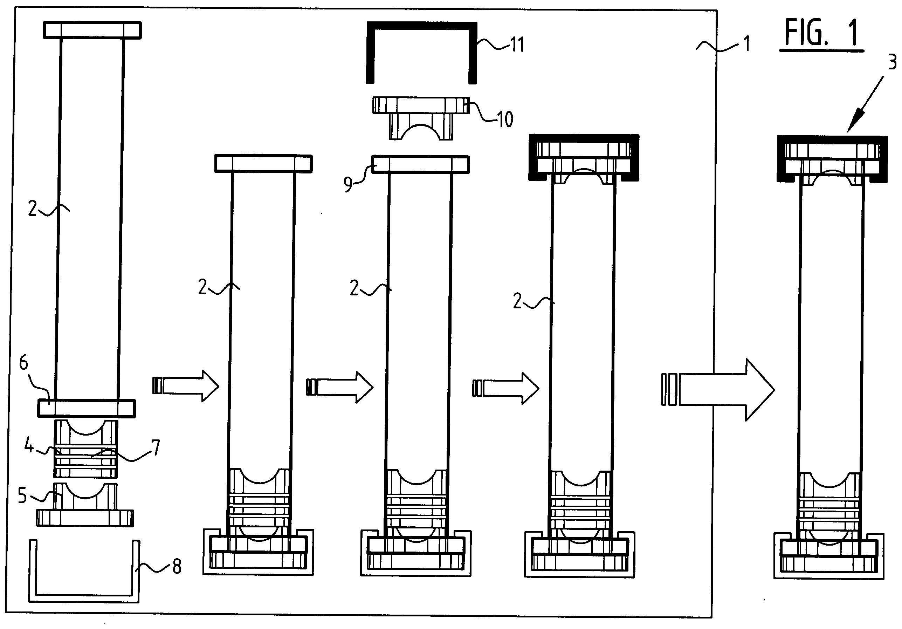

In fig. 1 a chamber 1 is shown, in which preferably an underpressure or vacuum is achieved, and in which a vial 2 is processed to manufacture a container 3 according to the present invention.

Before introducing the vial 2 into the vacuum chamber 1, it is appropriately cleaned. For instance, the vial can, prior to introducing it into the vacuum chamber 1, be cleaned using a method, that is conventional in medicine, such as gamma sterilisation.

After introducing the vial 2 into the vacuum chamber 1, subsequently a plunger 4 and an exit septum 5 are introduced into the opening at the side 6 of the vial 2, that is in a later stage intended to form the distal side 6 of the container 3.

The plunger 4 itself contains a septum 7.

After introducing the plunger 4 and the exit septum 5 into the opening at the distal side 6 of the vial 2, a crimp cap (aid) is arranged over the exit septum 5 and attached to the distal side 6 of the vial 2. Thereafter, at the other side 9 of the vial 2, where the vial 2 also exhibits an opening, and which side 9 is a later stage intended to form the proximal control side of the container 3, a closure septum 10 is introduced. Over the closure septum 10 another cap 11 is thereafter arranged to fixate the closure septum 10 at the opening at the proximal control side 9 of the vial 2. Caps 11, 8 also are used for protecting the interior of the vial 2 against contamination.

After having arranged the cap 11 over the closure septum 10, thus obtained container 3 according to the present invention can be taken out of the vacuum chamber and stored, prior to use. Before storage, by way of an option, the container 3 can be subjected to gamma sterilisation.

Needless to say, the sequence of steps described here and above can be reversed, and the closure septum 10 could be introduced into the opening at the proximal control side 9 of the vial 2 first, followed by the plunger 4 and the exit septum 5 at the distal side 6 of the vial 2, or this could be achieved practically simultaneously. Thereafter in a random order the caps 8, 11 can be attached over the septa 5 and 10. Any arbitrary order will suffice in order to obtain a container 3 as an embodiment of the present invention.

It is further noted that the caps 8, 11 can be colour coded to indicate the respective sides intended for introducing saline solution and active material and to thus prevent erroneous connection of the assembly components.

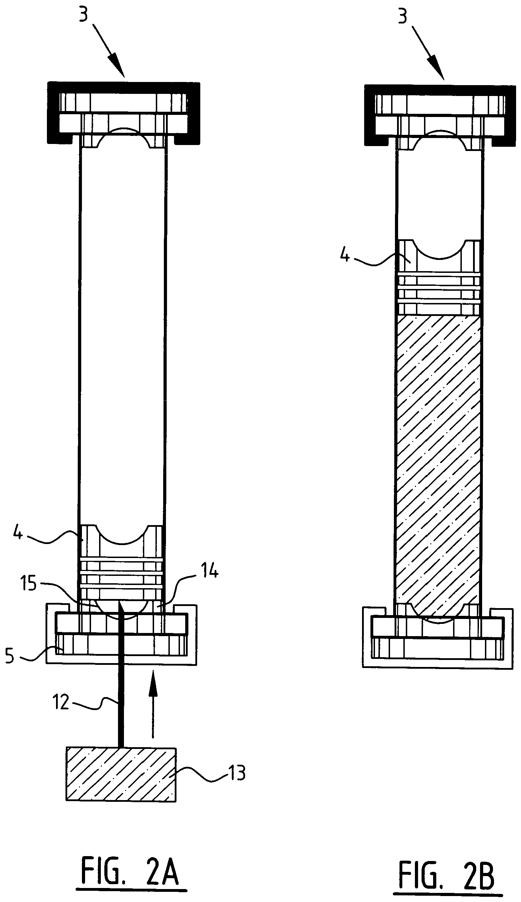

In fig. 2A and fig. 2B is shown, how further preparations for use are performed on a container 3 according to the present invention, in particular the container 3, which is the end result of the steps, that are schematically shown in fig. 1. In fig. 2A a needle 12 is introduced through the exit septum 5 to a position to open out into the space in between the movable plunger 4 and the exit septum 5. After having achieved the desired positioning of the needle 12, active material 13 can be introduced into said space between the movable plunger 4 and the exit septum 5, as a result of which the movable plunger 4 is lifted to the position shown in fig. 2B. Thereafter, the needle 12 is withdrawn.

In order to insure that the needle 12 can be introduced easily into a space between the movable plunger 4 and the exit septum 5, spacers 14 can be arranged between the movable plunger 4 and the exit septum 5. Such spacer 14 can in a circular manner enclose the afore mentioned space, thus enabling introduction of the needle 12 into said space 15. Quite possibly, a stop can be arranged on the needle to forward the needle 12 over at most such a distance through the septum 5, that the needle 12 is introduced no further than into the space 15. As an alternative, the length of the needle 12 can be chosen such that it does not extend beyond the space 15, when fully introduced through the exit septum 5.

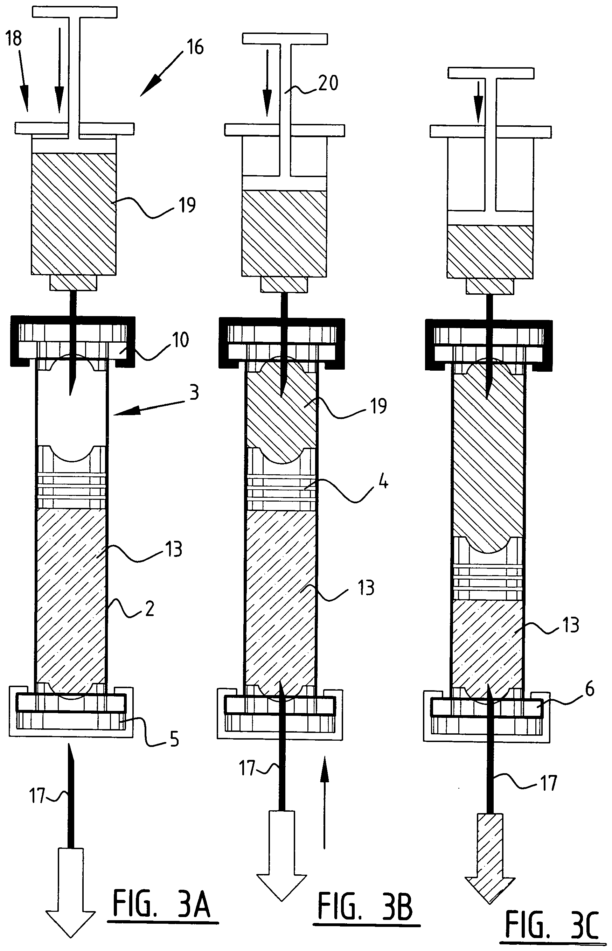

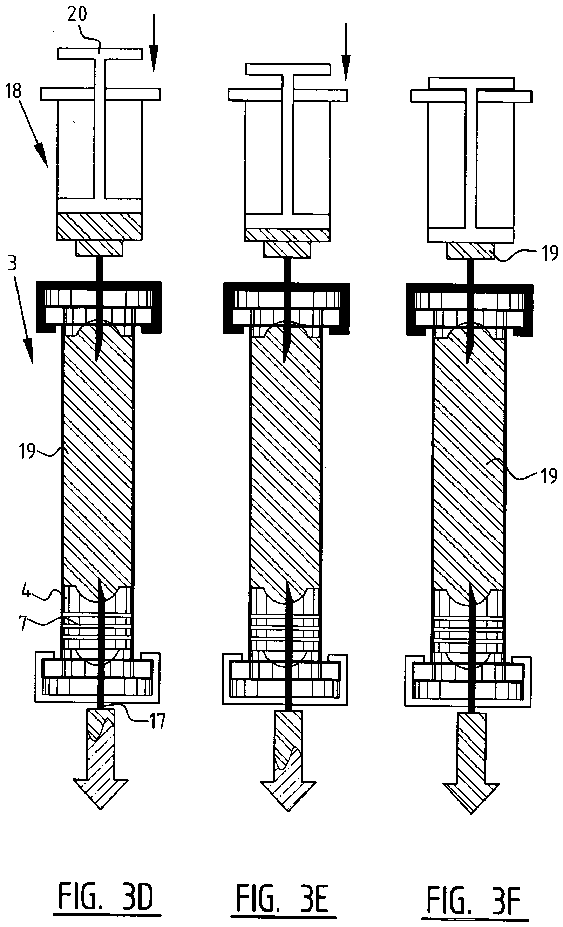

In fig. 3A-3F the container 3 of figures 1 and 2 is shown in cooperation with other components, together making up an assembly according to the present invention. The assembly has for a primary purpose the administration to a patient of a dose of active material 13, for example a radio active substance containing fluid or a contrast medium fluid for imaging purposes. Exemplary active material 13 has been introduced into the container 3 beforehand, as shown in fig. 2A and fig. 2B.

To assemble the assembly 16, a needle 17, which is connected to a conduit configuration (not shown) leading to the patient, is introduced into the interior of the vial 2 through the exit septum 5, as shown in fig. 3B. However, first the following steps are performed. A syringe 18 filled with a saline solution 19 was introduced through the closure septum 10 at the proximal control side of the container 3, as shown in fig. 3A. Then, as shown in fig. 3B, a portion of the saline fluid 19 from the syringe 18 was introduced into the vial 2 in the compartment opposite the compartment filled with active material 13 relative to the movable plunger 4. Thus it is prevented that the vacuum (see the description to figures) in said compartment, intended for the saline solution, causes the withdrawal of the fluids from the conduit configuration and thus unintentionally also from the patient, which could occur if the needle 17 were introduced first in stead of the preferred sequence wherein the syringe 18 is introduced first. As a result the container 3 is then filled with active material 13 on the one hand and saline solution 19 on the other hand, each in a separate compartment, where the compartments in the vial 2 are separated by the movable plunger 4.

Thereafter, needle 17 is introduced on the direction indicated with the solid arrow in fig. 3B.

If there is not a vacuum in the interior of the container 3 prior to filling, venting needles should be introduced in the steps depicted in figures 2A and 3B for filing the container 3.

When the push rod 20 of the syringe 18 is depressed further, as shown in fig. 3C, the pressure in the container 3 on the side of the saline fluid 19 increases as a result of which the plunger 4 is urged downward into the direction of the exit side 6 of the container 3. Also, as a result active material is ejected (indicated by dashed lines) on the open arrow at the needle 17, through the needle 17 to pass along the conduit configuration (not shown), of which needle 17 forms a part, and into the patient to be treated. Such treatment is for example an imaging treatment, for which the active material is to be introduced into the vascular system of the patient.

When the amount of active material 13 in the container 3 is just about depleted or injected, the plunger 4 comprising septum 7 moves over the needle 17 and the septum 7 is pierced thereby. Still at this time the control 20 of the syringe 18 is depressed further and further, as a result of which the needle 17, which is than in open contact with the part of the container 3 with the saline solution 19 therein, is flushed or rinsed with that saline solution 19 (indicated by the double line batching in the open arrow at needle 17). This is to say, that the possibly radio active or otherwise active material, remaining as a residue in the needle 17 and the further conduits leading to the patient, are flushed away with saline fluid, which does not have a harmful effect, when introduced into the patient also. Thus, the system can be cleaned by flushing or rinsing, which is in particular shown in fig. 3E. Thereafter the control 20 of the syringe 18 will ultimately also reach its final position, in which practically all of the saline solution 19 will have been driven out of the syringe 18. At such time the plunger 4 in the container 3 will still have been pierced by the needle 17, but as a result of a subsequent drop inn pressure in the saline solution 19 in the container 3 no further flushing of the needle 17 or the subsequent conduits will occur. Therefore, it is of importance to use, in the assembly as shown in figures 3A-3F, a syringe 18 with a sufficient volume capacity to introduce enough of the saline solution 19 to effectively flush or rinse out any and all of the residual active material from at least the needle 17 and the subsequent conduits towards the patient.

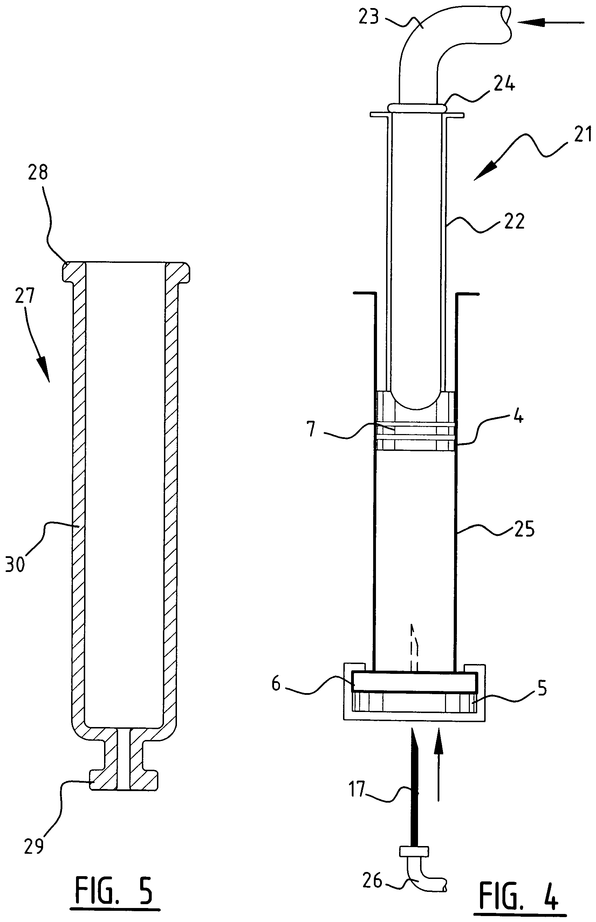

The previous description has been drafted, referring to the enclosed drawings of figures 1-3. However, in these figures essentially a single embodiment of the container 3 has been shown. Fig. 4 in contrast relates to an alternative container 20 which is not according to the invention. The container 20 resembles to a higher degree a conventional syringe, accept for the presence of an exit septum 5 at the distal side 6 of the container 20, where conventionally a needle would normally have been attached instead of the exit septum 5.

Relative to the previous embodiment of figures 1-3 the needle 17 is therefore still also the same. However, for providing a saline fluid supply an alternative is used in the embodiment of fig. 4. In fig. 4 the plunger 4 is attached to a hollow push rod 22, resembling a conventional push control of a normal syringe, but in this case the control rod 22 is, as mentioned herein before, hollow.

Further, a conduit or tube 23 is connected at connector 24 to the hollow interior of the push rod 22. The arrow in fig. 4 indicates the direction, in which needle 17 is to be inserted into the lower compartment with the saline fluid can be provided.

The plunger 4 can be promoted in the direction of the distal end 6 of the container 21 manually or by arranging a drive in some manner between the hollow push rod 22 and the housing 25 of the container 21.

When the plunger 4 is moved in the direction of the distal side 6 of the container 21 to have driven out practically all of the active material, the septum 7 in the plunger 4 will again be pierced by the needle 17, that is introduced through the exit septum 5, as shown in dashed lines in fig. 4. Thereafter, the open end of the needle 17 is in open contact with the interior of the hollow push rod 22, that is connected to the plunger 4. Thereafter, a free passage of saline fluid (or any other possible flushing fluid) can pass through the needle 17 and through the conduit configuration to remove residues of the active material from the interior of the needle 17 and the subsequent conduit configuration. 26.

In another embodiment, as shown in fig. 5, of a container 27 according to the present invention, the proximal side 28 can have a different shape or diameter than the distal side 29 of the vial 30 making up the container. This conserve the purpose as already indicated herein above in relation to fig. 1, where the closure caps 8, 11 can have different colours for coding which side (proximal 9 or distal 6) of the container 3 is intended for accommodating e.g. a radio active substance containing fluid and/or a contrast medium fluid, or saline solution fluid. Correspondingly, in the embodiment of fig. 5, the proximal side 28 is wider than the distal side 29, and as such the smaller diameter distal side 29 indicates that the radio active substance containing fluid and/or a contrast medium fluid should be accommodated on the corresponding side of the container 27.

After taking note of the previous description of embodiments of the present invention, the skilled person will be compelled to contemplate further embodiments, that all lie within the scope of protection, provided by the appended claims.

In yet another possible use in combination with active material, e.g. radio active substance containing fluid and/or a contrast medium fluid, a container can be formed as a multi-dose device, which is able to deliver subsequent partial doses of any fluid accommodated therein, that needs to be administered carefully in pre-defined dosages. For flushing or rinsing conduit configurations, that are subsequently connected to such a container, a separate flushing system or supply can be provided, which can than be attached or attachable to the output at the distal side of the container. However, when a plunger is used and it reaches the ultimate position thereof in the container, than preferably a channel or conduit is opened for flushing fluid at or from the proximal side of the container. However, also when the plunger has reached the ultimate position thereof within the container, a flushing system may also be provided for at the output on the distal side of the container, which can than form an additional and possibly preferable, though not restricted, element in the assembly according to the present invention.

## Claims

1. A container in or for an assembly for administering to a patient a dose of active material, the container comprising:
- a distal ejection side with an exit comprising an exit septum, which is piercable or at least in use pierced by a needle, and a proximal control side (9); and
- a plunger, being selectively movable, at least
from the proximal to the distal side of the container to eject the dose, which is pre-filled in the container, from the exit, where the exit of the container is connectable or at least in use connected to a conduit configuration, which is further connectable or at least in use connected to the patient, wherein the plunger comprises a passage septum for a flushing fluid, which passage septum is normally closed and arranged to be selectively opened by an opener comprising a needle, which is arrangable or at least in use is arranged at the exit to act on the plunger to open the passage septum with the plunger in a position in proximity of the exit,
**characterized in that** the proximal control side comprising a closure septum (10), the control side connectable or at least in use connected to a flushing fluid supply, wherein a cap (11) is arranged over the closure septum (10) to fixate the closure septum at the opening at the proximal control side, and **in that** the plunger is movable by the flushing fluid.

2. An assembly for administering to a patient a dose of active material, e.g. a radio active substance containing fluid or a contrast medium fluid for imaging purposes, the assembly comprising:
- a container, according to clam 1,
- a conduit configuration, which is connectable or at least in use connected to the exit of the container on the one hand and to the patient on the other hand, and
- an opener, which is arrangable or at least in use arranged at the exit to act on the plunger to open the passage with the plunger moved to a position in proximity of the exit, wherein the opener comprises a needle and the passage comprises a passage septum for selective piercing by the opener needle.

3. Assembly as defined in claim 2, wherein a single needle is arrangable or at least in use arranged to pierce both the exit septum and the passage septum.

4. Assembly as defined in claim 3, wherein the single needle is connected to the conduit configuration.

5. Assembly as defined in claims 2, 3 and 4, comprising a flushing fluid supply, wherein the flushing fluid supply comprises a needle for piercing or at least in use actually piercing the closure septum.

6. Assembly as defined in claims 2-5, wherein the flushing fluid supply comprises a pressure exerting component, such as a flushing fluid filled syringe, for selectively inserting flushing fluid into the container at the proximal control side thereof and for thereby selectively moving the plunger.

## Patentansprüche

1. Behälter in einer oder für eine Anordnung zum Darreichen einer Dosis aktiven Materials an einen Patienten, wobei der Behälter umfasst:
- eine distale Ausgabeseite mit einem Ausgang mit einer Ausgangswand, die durchdringbar oder wenigstens in Gebrauch über eine Nadel durchstoßen ist, und einer proximalen Steuerseite (9); und
- einen Kolben, der selektiv bewegbar ist, wenigstens von der proximalen zu der distalen Seite des Behälters, um die Dosis auszugeben, die in den Behälter vorgefüllt ist, von dem Ausgang, wo der Ausgang des Behälters mit einer Leitungskonfiguration verbindbar ist oder wenigstens in Gebrauch mit einer Leitungskonfiguration verbunden ist, die weiter mit dem Patienten verbindbar ist oder wenigstens in Gebrauch mit dem Patienten verbunden ist, wobei der Kolben eine Durchgangswand für ein durchströmendes Fluid umfasst, wobei die Durchgangswand normalerweise geschlossen ist, und angeordnet ist, um selektiv über einen Öffner mit einer Nadel geöffnet zu werden, die an dem Ausgang anordenbar ist oder wenigstens in Gebrauch an dem Ausgang angeordnet ist, um auf den Kolben zu wirken, um die Durchgangswand mit dem Kolben in einer Position in einer Nähe des Ausgangs zu öffnen,
**dadurch gekennzeichnet, dass** die proximale Steuerseite eine Schließwand (10) umfasst, die Steuerseite mit einer Zufuhr von durchströmenden Fluid verbindbar ist oder wenigstens in Gebrauch mit einer Zufuhr von durchströmenden Fluid verbunden ist, wobei ein Deckel (11) über der Schließwand (10) angeordnet ist, um die Schließwand an der Öffnung an der proximalen Steuerseite zu fixieren, und dadurch, dass der Kolben über das durchströmenden Fluid bewegbar ist.

2. Anordnung zum Darreichen einer Dosis eines aktiven Materials, beispielsweise einer radioaktiven Substanz mit einem Fluid oder einem Kontrastmittelfluid für Bildgebungszwecke, an einen Patient, wobei die Anordnung umfasst
- einen Behälter gemäß Anspruch 1,
- eine Leitungskonfiguration, die mit dem Ausgang des Behälters auf der einen Seite und mit dem Patienten auf der anderen Seite verbindbar ist oder wenigstens in Gebrauch verbunden ist, und
- ein Öffnungsmittel, das an dem Ausgang anordenbar ist oder wenigstens in Gebrauch an dem Ausgang angeordnet ist, um auf den Kolben zu wirken, um den Durchlass zu öffnen, mit dem Kolben in eine Position in einer Nähe des Ausgangs bewegt, wobei das Öffnungsmittel eine Nadel aufweist und der Durchlass eine Durchgangswand für ein selektives Durchdringen über die Öffnungsnadel umfasst.

3. Anordnung gemäß Anspruch 2, bei der eine Einzelnadel anordenbar ist oder wenigstens in Gebrauch angeordnet ist, um beide, die Ausgangswand und die Durchgangswand, zu durchdringen.

4. Anordnung gemäß Anspruch 3, bei der die Einzelnadel mit der Leitungskonfiguration verbunden ist.

5. Anordnung gemäß Anspruch 2, 3 und 4, umfassend ein Zufuhr für strömendes Fluid, wobei die Zufuhr für strömendes Fluid eine Nadel zum Durchdringen der Schließwand oder wenigstens in Gebrauch zum tatsächlich Durchdringen der Schließwand umfasst.

6. Anordnung gemäß Ansprüchen 2 bis 5, bei der die Zufuhr für strömendes Fluid einen druckausübenden Bestandteil umfasst, wie beispielsweise eine mit strömenden Fluid gefüllte Spritze, für eine selektive Einführung von strömenden Fluid in den Behälter an der proximalen Steuerseite davon und um dadurch selektiv den Kolben zu bewegen.

## Revendications

1. Récipient dans ou pour un ensemble destiné à administrer une dose de matière active, à un patient, le récipient comprenant :
un côté d'éjection distal avec une sortie comprenant un septum de sortie, qui peut être percé ou au moins, à l'usage, percé par une aiguille, et un côté de commande proximal (9) ; et
un piston plongeur, qui est sélectivement mobile, au moins du côté proximal au côté distal du récipient afin d'éjecter la dose, qui est pré-remplie dans le récipient, à partir de la sortie, où la sortie du récipient peut être raccordée ou au moins, à l'usage, raccordée à une configuration de conduit, qui peut en outre être raccordée ou au moins, à l'usage, raccordée au patient, dans lequel le piston plongeur comprend un septum de passage pour un fluide de rinçage, lequel septum de passage est normalement fermé et agencé pour être sélectivement ouvert par un dispositif d'ouverture comprenant une aiguille, qui peut être agencée ou au moins, à l'usage, est agencée à la sortie pour agir sur le piston plongeur afin d'ouvrir le septum de passage avec le piston plongeur dans une position à proximité de la sortie,
**caractérisé en ce que** le côté de commande proximal comprend un septum de fermeture (10), le côté de commande pouvant être raccordé ou au moins, à l'usage, raccordé à une alimentation en fluide de rinçage, dans lequel un capuchon (11) est agencé sur le septum de fermeture (10) pour fixer le septum de fermeture au niveau de l'ouverture, du côté de commande proximal, et **en ce que** le piston plongeur peut être déplacé par le fluide de rinçage.

2. Ensemble pour administrer une dose de matière active, à un patient, par exemple une substance radioactive contenant un fluide ou un fluide de milieu de contraste à des fins d'imagerie, l'ensemble comprenant :
un récipient selon la revendication 1,
une configuration de conduit qui peut être raccordée ou au moins, à l'usage, raccordée à la sortie du récipient d'une part et au patient d'autre part, et
un dispositif d'ouverture qui peut être agencé ou au moins, à l'usage, agencé à la sortie pour agir sur le piston plongeur afin d'ouvrir le passage avec le piston plongeur déplacé dans une position à proximité de la sortie, dans lequel le dispositif d'ouverture comprend une aiguille et le passage comprend un septum de passage pour le perçage sélectif par l'aiguille du dispositif d'ouverture.

3. Ensemble selon la revendication 2, dans lequel une seule aiguille peut être agencée ou au moins, à l'usage agencée pour percer à la fois le septum de sortie et le septum de passage.

4. Ensemble selon la revendication 3, dans lequel l'aiguille unique est raccordée à la configuration de conduit.

5. Ensemble selon les revendications 2, 3 et 4, comprenant une alimentation en fluide de rinçage, dans lequel l'alimentation en fluide de rinçage comprend une aiguille pour percer ou au moins, à l'usage percer vraiment le septum de fermeture.

6. Ensemble selon les revendications 2 à 5, dans lequel l'alimentation en fluide de rinçage comprend un composant exerçant une pression, telle qu'une seringue remplie de fluide de rinçage, pour insérer sélectivement de fluide de rinçage dans le récipient au niveau de son côté de commande proximal et pour sélectivement déplacer ainsi le piston plongeur.
